(19) Europäisches Patentamt
European Patent Office
Office européen des brevets

(11) Veröffentlichungsnummer: **0 046 243**
**B1**

(12) # EUROPÄISCHE PATENTSCHRIFT

(45) Veröffentlichungstag der Patentschrift: **11.07.84**

(51) Int. Cl.³: **A 61 K 31/53**, C 07 D 405/06 // C07D251/34

(21) Anmeldenummer: **81106158.9**

(22) Anmeldetag: **06.08.81**

(54) **Cytostatisch wirkende Arzneimittel, neue Isocyanursäurederivate und Verfahren zu ihrer Herstellung.**

(30) Priorität: **14.08.80 AT 4169/80**

(43) Veröffentlichungstag der Anmeldung: **24.02.82 Patentblatt 82/8**

(45) Bekanntmachung des Hinweises auf die Patenterteilung: **11.07.84 Patentblatt 84/28**

(84) Benannte Vertragsstaaten: **DE**

(56) Entgegenhaltungen:
**DE - A - 1 954 531**
**DE - A - 2 907 349**
**GB - A - 941 507**
**GB - A - 1 439 432**
**GB - A - 2 060 633**

**COMPTES RENDUES DES SEANCES DE L'CADEMIE DES SCIENCES, Band 281, 1975, Série C, Seiten 275-278, Paris, FR. A. ETIENNE et al.: "Isocyanurates de diallyle-1.3 et d'aryle-5"**
**ANGEWANDTE CHEMIE, 80, Jahrgang, 21. Oktober 1968, Nr. 20, Seiten 851-852 M. BUDNOWSKI: "Herstellung und Eigenschaften der diastereomeren Triglycidylisocyanurate"**
**CHEMICAL ABSTRACTS, Band 90, 1979, Seite 33, Nr. 138652d, Columbus, Ohio, U.S.A.**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Zeidler, Ulrich, Dr., Heinrich-Lersch-Strasse 19, D-4000 Düsseldorf (DE)**
Erfinder: **Fischer, Herbert, Dr., Kamper Weg 139, D-4000 Düsseldorf 12 (DE)**
Erfinder: **Hase, Brigitte, Dr., Millrather Weg 29, D-4006 Erkrath 1 (DE)**
Erfinder: **Möller, Hinrich, Dr., Schumannstrasse 11, D-4019 Monheim (DE)**
Erfinder: **Wilk, Hans-Christoph, Dr., An der Obererft 94, D-4040 Neuss (DE)**

(56) Entgegenhaltungen: (Fortsetzung)
**CHEMICAL ABSTRACTS, Band 89, 1978, Seite 44, Nr. 19857z, Columbus, Ohio, U.S.A.**
**CHEMICAL ABSTRACTS, Band 81, 1974, Seite 69, Nr. 137540t, Columbus, Ohio, U.S.A.**

**EP 0 046 243 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, dass eine Reihe von alkylierenden Substanzen eine cytostatische beziehungsweise cytotoxische Wirkung entfalten. Die bekanntesten Verbindungen leiten sich vom sogenannten Stickstofflost ab. Darüber hinaus ist es auch bekannt, wenigstens zwei Epoxidgruppen im Molekül enthaltende Verbindungen als Cancerostatika zu verwenden. Derartige Verbindungen sind beispielsweise das 4,4'-Bis-(2,3-epoxypropyl)-di-piperidinyl-(1,1') und das 1,2-15,16-Diepoxy-4,7-10,13-tetraoxohexadecan. Allerdings haben die letzteren Verbindungen keine wesentliche Verbesserung in der cytostatischen Behandlung gebracht und werden kaum verwendet. Lediglich zur Behandlung von Hirntumoren werden sie noch gelegentlich eingesetzt. Einer breiteren Anwendung steht auch die begrenzte Löslichkeit der erwähnten Verbindungen entgegen.

Gegenstand der europäischen Patentschrift Nr. 14 981 sind Arzneimittelzubereitungen mit cytostatischer Wirksamkeit, die als pharmakologischen Wirkstoff Triglycidylisocyanurat (TGI) und/oder solche TGI-Derivate enthalten, in denen das Wasserstoffatom des Kohlenstoffs in 2-Stellung der Glycidylgruppe durch einen Alkylrest mit 1 bis 4 Kohlenstoffatomen ersetzt sein kann. Verbindungen dieser Art zeichnen sich dadurch aus, dass die drei N-Atome des Isocyanursäurerings mit Epoxygruppen enthaltenden Glycidylresten substituiert sind, die in 2-Stellung auch mit einem Alkylrest mit 1 bis 4 Kohlenstoffatomen substituiert sein können.

Die vorliegende Erfindung geht von der Feststellung aus, dass strukturanaloge Verbindungen ebenfalls eine überraschend starke cytostatische Wirksamkeit entfalten, die die Wirksamkeit von TGI sogar übertreffen kann. Die erfindungsgemäss beschriebenen und eingesetzten strukturanalogen Verbindungen enthalten am Isocyanursäurering 2 oder 3 Epoxidgruppen enthaltende Reste in N-Substitution, wobei wenigstens einer dieser N-Substituenten in seiner Struktur von der Glycidylgruppe aus der erwähnten älteren europäischen Patentschrift abweicht.

Gegenstand der vorliegenden Erfindung sind dementsprechend in einer ersten Ausführungsform Arzneimittelzubereitungen mit cytostatischer Wirksamkeit, enthaltend Verbindungen der allgemeinen Formel I

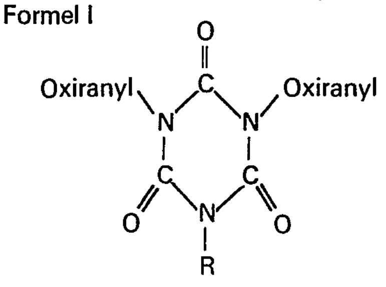

(I)

in der Oxiranyl gleiche oder verschiedene Reste der allgemeinen Formel II bedeutet

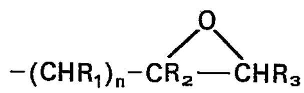

(II)

worin $R_1$, $R_2$ und $R_3$ gleich oder verschieden sind und Wasserstoff oder Alkyl, Halogenalkyl, Hydroxyalkyl und/oder Alkoxyalkyl mit jeweils 1 bis 4 Kohlenstoffatomen und n eine Zahl von 1 bis 8 mit der Massgabe bedeuten, dass im Falle, dass in allen Oxiranlyresten n=1 ist, mindestens einer der Reste $R_1$ oder $R_3$ ungleich Wasserstoff ist, und wobei weiterhin R in der allgemeinen Formel I einen Oxiranylrest der allgemeinen Formel II, Wasserstoff oder einen der folgenden Reste bedeutet: Alkyl, Alkenyl, Aryl, Aralkyl, Alkaryl oder Cycloalkyl mit maximal 12 Kohlenstoffatomen.

Gegenstand der Erfindung sind in weiteren Ausführungsformen die gemäss der vorstehenden Formel I gekennzeichneten neuen Verbindungen sowie die Verfahren zu ihrer Herstellung. Diese Herstellung erfolgt nach grundsätzlich an sich bekannten Verfahren und wird im folgenden noch geschildert.

Die erfindungsgemäss eingesetzten Verbindungen zeichnen sich dadurch aus, dass sie in N-Substitution am Isocyanursäurering wenigstens 2 Substituenten mit Epoxidgruppen enthalten, wobei weiterhin wenigstens einer der Reste von dem in der genannten europäischen Patentschrift Nr. 14 981 offenbarten Glycidylrest

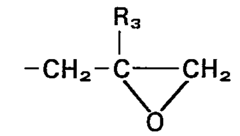

(III)

verschieden ist,
wobei in dieser Formel III $R_3$ bevorzugt Wasserstoff ist, aber auch niedere Alkylreste mit 1 bis 4 Kohlenstoffatomen bedeuten kann. Wenigstens in einem der erfindungsgemäss vorgesehenen Oxiranylreste in N-Substitution liegt – im Rahmen der erfindungsgemäss gegebenen Definition für diese Reste der allgemeinen Formel II – eine von den zuvor genannten Glycidylresten der Formel III abweichende Konstitution vor.

Es kann zweckmässig sein, entsprechende Verbindungen mit jeweils nicht mehr als 3 C-Atomen in diesen Resten $R_1$ bis $R_3$ einzusetzen, wobei entsprechenden Verbindungen besondere Bedeutung zukommt, die in diesen Resten jeweils 1 oder 2 C-Atome besitzen. Wegen ihrer Ähnlichkeit zu den hochwirksamen TGI-Derivaten der genannten europäischen Patentschrift können Verbindungen besonders bevorzugt sein, in denen diese Reste $R_1$ bis $R_3$ nur ein Kohlenstoffatom aufweisen, wobei es hier wiederum besonders bevorzugt sein kann, dass nicht alle, sondern nur 2 oder gar auch nur einer dieser Reste einen entsprechenden Alkylrest beziehungsweise substituierten Alkylrest bedeutet, während die verbleibenden Reste Wasserstoff sind. Diese Angaben haben besondere Bedeutung für den Fall, dass n in den Oxiranylresten der allgemeinen Formel II die Ziffer 1 bedeutet. Ist n eine von 1 abweichende Zahl, dann können in einer weiteren bevorzugten Ausführungsform alle Reste $R_1$ bis $R_3$ Wasserstoff sein. Ist wenigstens einer dieser Reste in einem solchen Fall ein gegebenenfalls substituierter Alkylrest, dann gelten sinngemäss die zuvor gemachten Angaben zur bevorzugten Beschaffenheit solcher Reste.

Bevorzugte Werte für n liegen im Zahlenbereich von 1 bis 5, insbesondere im Bereich von 1 bis 3. Verbindungen mit der Bedeutung n=2 oder insbesondere n=1 zählen zu besonders wichtigen Verbindungen im Sinne der Erfindung, wobei im zuletztgenannten Falle die zuvor angegebenen Einschränkungen zu berücksichtigen sind.

Zur bevorzugten Beschaffenheit der Reste $R_1$, $R_2$ und $R_3$ in den Oxiranylresten der allgemeinen Formel II gelten die folgenden Angaben: $R_1$ ist vorzugsweise Wasserstoff, ein Alkylrest oder ein substituierter Alkylrest mit 1 bis 3 C-Atomen. $R_2$ ist bevorzugt Wasserstoff, ein Alkylrest oder ein entsprechend substituierter Alkylrest mit einem C-Atom und $R_3$ ist vorzugsweise Wasserstoff, ein Alkylrest oder ein entsprechend substituierter Alkylrest mit 1 bis 2 C-Atomen. Allgemein gilt, dass Verbindungen, in denen $R_2$ Wasserstoff ist, besondere Bedeutung haben können und dass weiterhin zusätzlich auch Verbindungen mit $R_3$ = Wasserstoff bevorzugt sein können. Verbindungen dieser Art sind dadurch gekennzeichnet, dass die Epoxidgruppe in wenigstens einem oder vorzugsweise in beiden Kohlenstoffatomen des Epoxidringes unsubstituiert ist.

Der Wirkungsmechanismus der im Rahmen der Erfindung eingesetzten Verbindungen ist im einzelnen nicht geklärt. Offenbar sind es jedoch die Epoxidgruppen, die für die cytostatische Wirkung verantwortlich sind und die wenigstens zweifach im Molekül der erfindungsgemäss eingesetzten Verbindungen vorliegen. Möglicherweise liegt die Bedeutung des dritten Substituenten – der eine Epoxidgruppe enthalten kann aber nicht muss – im Einfluss auf die Verteilung von lipophilen und hydrophilen Präferenzen, so dass damit im gewissen Ausmass die Aufnahme der Verbindungen durch den Organismus gesteuert werden kann. Die Bedeutung dieses erfindungsgemäss eingeführten Substituenten R muss sich aber nicht auf diese Funktion beschranken.

Als vorteilhaft hat es sich allerdings auch erwiesen, die absolute Grösse der jeweiligen Oxiranylreste in N-Substitution einer gewissen Begrenzung zu unterwerfen. So weisen die bevorzugten Oxiranylreste insgesamt nicht mehr als 12 und dabei vorzugsweise nicht mehr als 10 Kohlenstoffatome auf. Es kann zweckmässig sein, Reste mit bis zu 8 oder gar nur mit bis zu 4 oder 5 Kohlenstoffatomen einzusetzen. Wie bereits ausgeführt, können 1 oder 2 Epoxidreste im Sinne der allgemeinen Formel III vorliegen, vorausgesetzt, dass wenigstens ein hiervon abweichender Oxiranylrest im Sinne der erfindungsgemässen Definition vorgesehen ist.

Der Rest R in den erfindungsgemäss eingesetzten Verbindungen der allgemeinen Formel I kann ein dritter Oxiranylrest im Sinne der erfindungsgemässen Definition, Wasserstoff oder ein Kohlenwasserstoffrest sein, insgesamt nicht mehr als 12 Kohlenstoffatome und dabei vorzugsweise nicht mehr als 8 Kohlenstoffatome. Interessant können insbesondere Reste sein, die bis zu 6 oder vorzugsweise sogar nur bis zu 4 Kohlenstoffatome enthalten, wobei diese Zahlenwerte unabhängig von der jeweiligen Struktur zu verstehen sind und sich auf die Summe aller Kohlenstoffatome im betroffenen Rest beziehen.

In einer besonders bevorzugten Ausführungsform der Erfindung bedeutet R einen Alkyl- oder Alkenylrest mit maximal 12 Kohlenstoffatomen. Dieser Alkyl- oder Alkenylrest kann geradkettig oder verzweigt sein und enthält vorzugsweise nicht mehr als 10, insbesondere nicht mehr als 8 Kohlenstoffatome. In dieser Ausführungsform der Erfindung sind insbesondere solche Verbindungen der allgemeinen Formel I bevorzugt, in denen der Rest R Alkyl mit 1 bis 6 C-Atomen oder einen entsprechenden Alkenylrest bedeutet.

Im Rahmen der Erfindung können Verbindungen der allgemeinen Formel I Verwendung finden, in denen der Rest R geradkettiges oder verzweigtes unsubstituiertes Alkyl mit bis zu 6, vorzugsweise mit bis zu 4 Kohlenstoffatomen bedeutet. In Betracht kommen insbesondere die Reste Methyl, Ethyl, Propyl, Isopropyl und die entsprechenden $C_4$-Reste.

Die Herstellung der erfindungsgemässen Verbindungen der allgemeinen Form I kann auf mehreren Wegen erfolgen. Hier gelten insbesondere die folgenden Angaben:

Grundsätzlich kann diese Herstellung in an sich bekannter Weise nach verschiedenen Wegen erfolgen, von denen die wichtigsten hier ohne Anspruch auf Vollständigkeit erwähnt seien.

Die Herstellung insbesondere von Triepoxyverbindungen der allgemeinen Formel I kann durch Umsetzung der Cyanursäure oder ihrer Salze mit den entsprechenden Alkenylhalogeniden und anschliessende Epoxidation der Alkenylgruppe erfolgen, vergleiche hierzu beispielsweise US-PS 3 376 301 und Houben-Weyl, «Methoden der organischen Chemie», Band 6/3, 385 und folgende.

Auch die Synthese über die Umsetzung von Cyanursäure mit α-Halogenepoxiden und folgende Halogenwasserstoffabspaltung ist möglich, vergleiche hierzu DE-OS 1 954 531 und «Angew. Chemie» 80 (1968), 851.

Für die Herstellung von Diepoxidverbindungen der allgemeinen Formel I gelten die folgenden Angaben: Auch ihre Herstellung kann in an sich bekannter Weise erfolgen. Dabei seien als Möglichkeiten aufgeführt:

Ein sehr elegantes allgemeines Verfahren zur Herstellung der Verbindungen der allgemeinen Formel I beruht auf der Umsetzung der mono-N-substituierten Cyanursäuren mit Epi-Halohydrinen. Die Herstellung von mono-N-substituierten Cyanursäuren kann nach literaturbekannten Verfahren erfolgen. Zur einschlägigen Literatur wird beispielsweise verwiesen auf W.J. Close, «J. Am. Chem. Soc.» 75 (1953) 3617. Beschrieben wird hier neben älteren einschlägigen Arbeiten ein allgemein anwendbares Verfahren, bei dem monosubstituierte Biuret-Verbindungen mit Alkylcarbonaten, insbesondere Ethylcarbonat, in Gegenwart von Alkalialkoxid, insbesondere Natriumethoxid, zur monosubstituierten Cyanursäure umgesetzt werden. Der auf diese Weise eingeführte Substituent entspricht dabei in der Regel dem Rest R aus den Verbindungen der allgemeinen Formel I. In nachfolgender Reaktion werden dann die beiden Oxiranylgruppen eingeführt. Hierzu setzt man in an sich bekannter Weise die monosubstituierte Cyanursäure mit der entsprechenden Epi-Halohydrinverbindung, beispielsweise mit Epi-Chlorhydrin um. Diese Umsetzung erfolgt ebenfalls in an sich bekannter Weise. Sie kann zum Beispiel in Gegenwart einer geringen Menge einer quartären Ammoniumverbindung als Katalysator durchgeführt werden (vergleiche hierzu beispielsweise Houben-Weyl, «Methoden der organischen Chemie», Band 14/2 (1963), 497, 547).

In einer Modifikation dieses Weges wird die monosubstituierte Cyanursäure nicht unmittelbar mit der Epoxidverbindung umgesetzt. Statt dessen erfolgt zunächst ihre Umsetzung mit Allylhalogeniden, die dem Oxiranylrest der Verbindungen der allgemeinen Formel I entsprechen, jedoch anstelle der Epoxidgruppe eine olefinische Doppelbindung aufweisen, woraufhin die entstandenen diallylsubstituierten Isocyanurate epoxidiert werden. Die Epoxidation kann in an sich bekannter Weise mit Persäuren vorgenommen werden. Die Umsetzung von Cyanursäure mit Allylhalogeniden ist beispielsweise beschrieben in der US-PS 3 376 301, die Epoxidation von Allylisocyanuraten mit Persäuren ist zum Beispiel in Houben-Weyl aaO, Band 6/3, 385 und folgende beschrieben. Typische Beispiele für Verbindungen der allgemeinen Formel I sind die folgenden:

1. Di-2.3-epoxybutyl-but-2-enyl-isocyanurat
   ($R = -CH_2-CH=CH-CH_3$, $R^1$, $R^2$ = H, $R^3 = -CH_3$, n = 1)
   Farblose, viskose Flüssigkeit
2. Tri-2.3-epoxybutyl-isocyanurat
   (R = 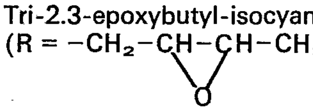 ; $R^1$, $R^2$ = H; $R^3 = -CH_3$; n = 1)
   Farblose, viskose Flüssigkeit
3. Tri-3.4-epoxybutyl-isocyanurat
   (R = 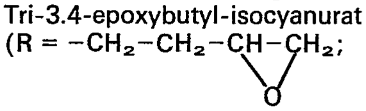 ; $R^1$, $R^2$, $R^3$ = H; n = 2)
   Farblose, viskose Flüssigkeit, nach mehrtägigem Stehen langsam kristallisierend
4. Tri-4.5-epoxypentyl-isocyanurat
   (R = 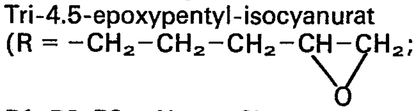 ; $R^1$, $R^2$, $R^3$ = H; n = 3)
   Farblose, viskose Flüssigkeit
5. Tri-5.6-epoxyhexyl-isocyanurat
   (R = 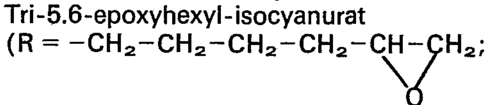 ; $R^1$, $R^2$, $R^3$ = H; n = 4)
   Farblose, viskose Flüssigkeit

Weitere Verbindungen, die in den Rahmen der Erfindung fallen sind beispielsweise:

Di-2.3-epoxybutyl-methyl-isocyanurat,
Di-2.3-epoxybutyl-butyl-isocyanurat,
Di-3.4-epoxybutyl-but-3-enyl-isocyanurat,
Di-4.5-epoxypentyl-pent-4-enyl-isocyanurat,
Di-5.6-epoxyhexyl-hex-5-enyl-isocyanurat,
Di-4.5-epoxy-pentyl-pentyl-isocyanurat,
Di-5.6-epoxyhexyl-hexyl-isocyanurat,
Di-3.4-epoxybutyl-butyl-isocyanurat,
Tri-(1-methyl-2.3-epoxy)propyl-isocyanurat,
Di-(1-methyl-2.3-epoxy)propyl-1-methyl-prop-2-enyl-isocyanurat,
Tri-2.3-epoxypentyl-isocyanurat,
Tri-3.4-epoxypentyl-isocyanurat,
Tri-2.3-epoxyhexyl-isocyanurat,
Tri-3.4-epoxyhexyl-isocyanurat,
Tri-4.5-epoxyhexyl-isocyanurat,
Tri-(2-methyl-2.3-epoxy)butyl-isocyanurat,
Tri-(4-methyl-4.5-epoxy)pentyl-isocyanurat,
Tri-(4-methyl-4.5-epoxy)hexyl-isocyanurat,
Tri-7.8-epoxyoctyl-isocyanurat.

Die erfindungsgemäss eingesetzten Verbindungen der allgemeinen Formel I treten in verschiedenen stereoisomeren Formen auf. Grundsätzlich eignen sich alle diese verschiedenen Formen für die Zwecke der Erfindung. Sie können dabei in Mischung oder auch in Form bestimmter isolierter Isomeren eingesetzt werden.

Zur Verwendung als Cancerostatika sollten die Wirksubstanzen mittels geeigneter Vehikel appliziert werden. Hier eignen sich die üblichen Hilfsbeziehungsweise Trägerstoffe für pharmakologische Zubereitungen.

Die erfindungsgemäss eingesetzten Verbindungen sind wirksam gegen verschiedene Leukämieformen sowie maligne Neoplasmen wie Lungencarcinom, Coloncarcinom, Melanome, Ependymoblastome und Sarcome. Eine Kombinationstherapie in Verbindung mit anderen alkylierenden Substanzen ist möglich.

*Beispiel 1*

*Darstellung des Tri-2.3-epoxybutyl-isocyanurats (Verbindung 2)*

29,6 g (0,152 mol) Trinatriumcyanurat und 42,1 g (0,456 mol) 1-Chlorbuten-2 wurden in 70 ml Dimethylformamid 3,5 h bei 120°C gerührt. Nach Abkühlung auf 100°C wurde mit 100 ml Toluol versetzt und mit 10%iger Kochsalzlösung behandelt. Die organische Phase wurde abgetrennt und das Lösungsmittel abdestilliert. Es blieben 42,3 g einer viskosen Flüssigkeit zurück. Kristallisation in Methanol ergab 24 g farblose Kristalle, Fp.: 77°C, 53% Ausbeute an Tri-but-2-enyl-isocyanurat.

5 g (0,017 mol) Tri-but-2-enyl-isocyanurat wurden in 15 ml Methylenchlorid gelöst, mit 10,9 g 90%iger m-Chlorperbenzoesäure (0,057 mol) versetzt, 2,5 h bei Raumtemperatur gerührt und über Nacht stehen gelassen. Die Oxidation ist leicht exotherm. Nach Beendigung der Reaktion wurde von der ausgefallenen m-Chlorbenzoesäure abfiltriert, eventuell noch vorhandene Persäure durch Schütteln mit Bisulfitlösung zerstört und die Säure mit wässeriger Hydrogencarbonatlösung extrahiert. Nach Destillation des Lösungsmittels verblieben 10,3 g eines flüssigen Produktgemischs, das säulenchromatographisch mit Kieselgel G (Firma Merck) und Methylenchlorid/Aceton 95/5 als Laufmittel aufgetrennt wurde. Es wurden 7,8 g (68%) des Tri-2.3-epoxybutyl-isocyanurats erhalten. Viskose, farblose Flüssigkeit mit 14,0% Epoxidsauerstoff.

*Beispiel 2*

*Darstellung des Tri-3.4-epoxybutyl-isocyanurats (Verbindung 3)*

9,8 g (0,05 mol) Trinatriumcyanurat und 20,4 g (0,15 mol) 1-Brombuten-3 wurden in 20 ml Dimethylformamid 6 h bei 120°C gerührt. Nach Aufarbeitung wie in Beispiel 1 wurde über Kieselgel filtriert. Nach Entfernung des Lösungsmittels verblieben 6,8 g einer dünnschichtchromatographisch einheitlichen Flüssigkeit, Jodzahl: 257, 6,8 g (46%) Tri-but-3-enyl-isocyanurat.

6,8 g (0,023 mol) Tri-but-3-enyl-isocyanurat und 22,2 g (0,116 mol) m-Chlorperbenzoesäure wurden in 300 ml Methylenchlorid 7 h bei 25 bis 30°C gerührt und über Nacht bei Raumtemperatur stehen gelassen. Nach Reduktion der überschüssigen Persäure, Ausschütteln der Säure und Destillation des Lösungsmittels verblieben 3,5 g einer viskosen Flüssigkeit mit 13,4% Epoxidsauerstoff. Das Produkt enthielt 95% Tri-2.3-epoxybutyl-isocyanurat. Ausbeute: 42%. Kristallisation nach mehrtägigem Stehen. Die Austestung der Verbindungen gemäss Beispiel 1 und Beispiel 2 nach Testvorschriften des National Cancer Institute Bethesda, Maryland 200014, veröffentlicht in «Cancer Chemotherapy Reports» Part. 3, September 1972, vol. 3, Nr. 2 liefert bei der Tumorart P 388 (Leukämie) eine signifikante Verlängerung der mittleren Überlebensdauer der behandelten Tiergruppe gegenüber der zum Vergleich eingesetzten unbehandelten Tiergruppe.

## Patentansprüche

1. Arzneimittelzubereitungen mit cytostatischer Wirksamkeit, enthaltend Verbindungen der allgemeinen Formel

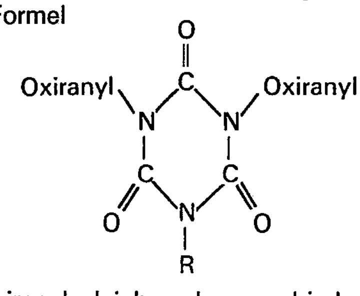

(I)

in der Oxiranyl gleiche oder verschiedene Reste der allgemeinen Formel bedeutet

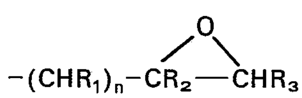

(II)

worin $R_1$, $R_2$ und $R_3$ gleich oder verschieden sind und Wasserstoff oder Alkyl, Halogenalkyl, Hydroxyalkyl und/oder Alkoxyalkyl mit jeweils 1 bis 4 Kohlenstoffatomen und n eine Zahl von 1 bis 8 mit der Massgabe bedeuten, dass im Falle, dass in allen Oxiranylresten n = 1 ist, mindestens einer der Reste $R_1$ und $R_3$ ungleich Wasserstoff ist, und wobei weiterhin R in der allgemeinen Formel I einen Oxiranylrest der allgemeinen Formel II, Wasserstoff oder einen der folgenden Reste bedeutet: Alkyl, Alkenyl, Aryl, Aralkyl, Alkaryl oder Cycloalkyl mit maximal 12 Kohlenstoffatomen.

2. Arzneimittelzubereitungen nach Anspruch 1, dadurch gekennzeichnet, dass sie Verbindungen der allgemeinen Formel I enthalten, in denen die Gesamtzahl der Kohlenstoffatome in jeweils einem Oxiranylrest der allgemeinen Formel II nicht mehr als 10, vorzugsweise nicht mehr als 8 beträgt.

3. Arzneimittelzubereitungen nach Ansprüchen 1 und 2, dadurch gekennzeichnet, dass sie Verbindungen der allgemeinen Formel I enthalten, in denen n im Oxiranylrest eine Zahl von 1 bis 5, vorzugsweise 1 bis 3 bedeutet.

4. Arzneimittelzubereitungen nach Ansprüchen 1 bis 3, dadurch gekennzeichnet, dass sie Verbindungen der allgemeinen Formel I enthalten, in denen wenigstens einer der Reste $R_1$ in den Oxiranylresten Wasserstoff oder Alkyl beziehungsweise substituiertes Alkyl mit 1 bis 3 C-Atomen bedeutet.

5. Arzneimittelzubereitungen nach Ansprüchen 1 bis 3, dadurch gekennzeichnet, dass sie Verbindungen der allgemeinen Formel I enthalten, in denen wenigstens einer der Reste $R_2$ in den Oxiranylresten Wasserstoff oder Alkyl beziehungsweise substituiertes Alkyl mit einem Kohlenstoffatom bedeutet.

6. Arzneimittelzubereitungen nach Ansprüchen 1 bis 3, dadurch gekennzeichnet, dass sie Verbindungen der allgemeinen Formel I enthalten, in denen wenigstens einer der Reste $R_3$ in den Oxiranylresten Wasserstoff oder Alkyl beziehungsweise substituiertes Alkyl mit 1 bis 2 Kohlenstoffatomen bedeutet.

7. Arzneimittelzubereitungen nach Ansprüchen 1 bis 6, dadurch gekennzeichnet, dass sie Verbindungen der Formel I enthalten, die in ihrem Rest R insgesamt nicht mehr als 10 Kohlenstoffatome, vorzugsweise nicht mehr als 8 Kohlenstoffatome aufweisen.

8. Arzneimittelzubereitungen nach Ansprüchen 1 bis 7, dadurch gekennzeichnet, dass sie die Verbindungen der Formel I in Abmischung mit üblichen Hilfs- und/oder Trägerstoffen enthalten.

9. Verwendung der Arzneimittel gemäss Ansprüchen 1 bis 8 zur Therapie maligner Neoplasien.

10. Neue Oxiranylisocyanursäureverbindungen der allgemeinen Formel I

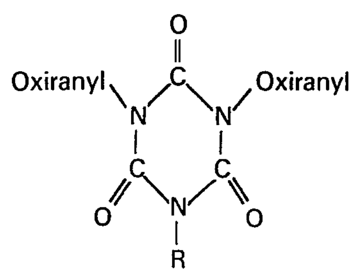

(I)

in der Oxiranyl gleiche oder verschiedene Reste der allgemeinen Formel II bedeutet

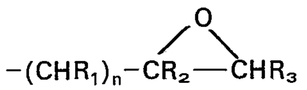

worin $R_1$, $R_2$ und $R_3$ gleich oder verschieden sind und Wasserstoff oder Alkyl, Halogenalkyl, Hydroxyalkyl und/oder Alkoxyalkyl mit jeweils 1 bis 4 Kohlenstoffatomen und n eine Zahl von 1 bis 8 mit der Massgabe bedeuten, dass im Falle, dass in allen Oxiranylresten n = 1 ist, mindestens einer der Reste $R_1$ oder $R_3$ ungleich Wasserstoff ist, und wobei weiterhin R in der allgemeinen Formel I einen Oxiranylrest der allgemeinen Formel II, Wasserstoff oder einen der folgenden Reste bedeutet: Alkyl, Alkenyl, Aryl, Aralkyl, Alkaryl oder Cycloalkyl mit max. 12 Kohlenstoffatomen.

## Claims

1. Cytostatic medicinal preparations containing compounds corresponding to the following general formula

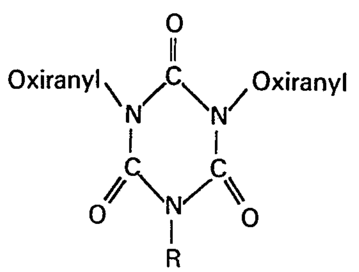

(I)

in which oxiranyl represents identical or different radicals corresponding to the following general formula

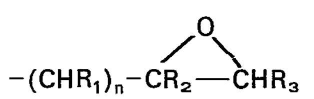

(II)

in which $R_1$, $R_2$ and $R_3$ are the same or different and represent hydrogen or alkyl, halogenalkyl, hydroxyalkyl and/or alkoxyalkyl each containing from 1 to 4 carbon atoms and n is a number of from 1 to 8, with the proviso that, where n = 1 in all the oxiranyl radicals, at least one of the radicals $R_1$ and $R_3$ is not hydrogen, and R in general formula I represents an oxiranyl radical corresponding to general formula II, hydrogen or one of the following radicals: alkyl, alkenyl, aryl, aralkyl, alkaryl or cycloalkyl containing at most 12 carbon atoms.

2. Medicinal preparations as claimed in Claim 1, characterized in that they contain compounds corresponding to general formula I in which the total number of carbon atoms in one oxiranyl radical corresponding to general formula II amounts to no more than 10 and preferably to no more than 8.

3. Medicinal preparations as claimed in Claims 1 and 2, characterized in that they contain compounds corresponding to general formula I in which n in the oxiranyl radical is a number of from 1 to 5 and preferably from 1 to 3.

4. Medicinal preparations as claimed in Claims 1 to 3, characterized in that they contain compounds corresponding to general formula I in which at least one of the radicals $R_1$ in the oxiranyl radicals is hydrogen or alkyl or substituted alkyl containing from 1 to 3 C-atoms.

5. Medicinal preparations as claimed in Claims 1 to 3, characterized in that they contain compounds corresponding to general formula I in which at least one of the radicals $R_2$ in the oxiranyl radicals is hydrogen or alkyl or substituted alkyl containing one carbon atom.

6. Medicinal preparations as claimed in Claims 1 to 3, characterized in that they contain compounds corresponding to general formula I in which at least one of the radicals $R_3$ in the oxiranyl radicals represents hydrogen or alkyl or substituted alkyl containing 1 to 2 carbon atoms.

7. Medicinal preparations as claimed in Claims 1 to 6, characterized in that they contain compounds corresponding to general formula I which, in their radical R, contain in all no more than 10 carbon atoms and preferably no more than 8 carbon atoms.

8. Medicinal preparations as claimed in Claims 1 to 7, characterized in that they contain the compounds of general formula I in admixture with standard auxiliaries and/or excipients.

9. The use of the medicinal preparations claimed in Claims 1 to 8 for treating malignant neoplasias.

10. New oxiranyl isocyanuric acid compounds corresponding to the following general formula

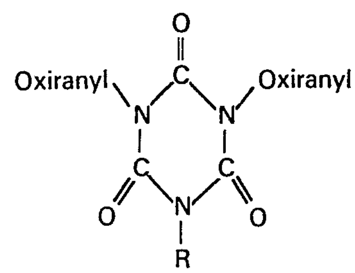

(I)

in which oxiranyl represents identical or different radicals corresponding to the following general formula II

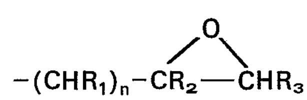

in which $R_1$, $R_2$ and $R_3$ are the same or different and represent hydrogen or alkyl, halogenalkyl, hydroxyalkyl and/or alkoxyalkyl each containing from 1 to 4 carbon atoms and n is an integer of from 1 to 8, with the proviso that, where n = 1 in all the oxiranyl radicals, at least one of the radicals $R_1$ or $R_3$ is not hydrogen, and R in general formula I is an oxiranyl radical corresponding to general formula II, hydrogen or one of the following radicals: alkyl, alkenyl, aryl, aralkyl, alkaryl or cycloalkyl containing at most 12 carbon atoms.

## Revendications

1. Préparations médicamenteuses à activité cytostatique, contenant des composés de formule générale:

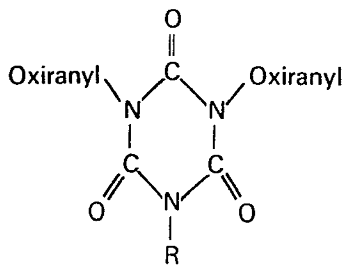

(I)

dans laquelle l'oxiranyle signifie des radicaux identiques ou différents de formule générale:

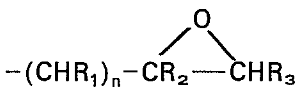

(II)

dans laquelle $R_1$, $R_2$ et $R_3$ sont identiques ou différents et signifient de l'hydrogène ou un alcoyle, halogénoalcoyle, hydroxyalcoyle et/ou alcoxyalcoyle contenant chaque fois 1 à 4 atomes de carbone et n est un nombre de 1 à 8, avec comme restriction qu'au cas où, dans tous les radicaux oxiranyle, n est égal à 1, au moins un des radicaux $R_1$ et $R_3$ ne soit pas de l'hydrogène et qu'en outre R, dans la formule générale I, signifie un radical oxiranyle de formule générale II, de l'hydrogène ou un des radicaux suivants: alcoyle, alcényle, aryle, aralcoyle, alcaryle ou cycloalcoyle ayant un maximum de 12 atomes de carbone.

2. Préparation médicamenteuses selon la revendication 1, caractérisées en ce qu'elles contiennent des composés de formule générale I, dans lesquels le nombre total des atomes de carbone, dans chaque radical oxiranyle de formule générale II, ne s'élève pas à plus de 10, de préférence pas à plus de 8.

3. Préparations médicamenteuses selon les revendications 1 et 2, caractérisées en ce qu'elles contiennent des composés de formule générale I, dans lesquels n, dans le radical oxiranyle, signifie un nombre de 1 à 5, de préférence de 1 à 3.

4. Préparations médicamenteuses selon les revendications 1 à 3, caractérisées en ce qu'elles contiennent des composés de formule générale I, dans lesquels au moins un des radicaux $R_1$, dans les radicaux oxiranyle, signifie de l'hydrogène ou un alcoyle, ou respectivement un alcoyle substitué ayant 1 à 3 atomes de carbone.

5. Préparations médicamenteuses selon les revendications 1 à 3, caractérisées en ce qu'elles contiennent des composés de formule générale I, dans lesquels au moins un des radicaux $R_2$, dans les radicaux oxiranyle, signifie de l'hydrogène ou un alcoyle, ou respectivement un alcoyle substitué avec un atome de carbone.

6. Préparations médicamenteuses selon les revendications 1 à 3, caractérisées en ce qu'elles contiennent des composés de formule générale I, dans lesquels au moins un des radicaux $R_3$, dans les radicaux oxiranyle, signifie de l'hydrogène ou un alcoyle, ou respectivement un alcoyle substitué ayant 1 à 2 atomes de carbone.

7. Préparations médicamenteuses selon les revendications 1 à 6, caractérisées en ce qu'elles contiennent des composés de formule I qui, dans leur radical R, ne présentent pas plus en tout que 10 atomes de carbone, de préférence pas plus de 8 atomes de carbone.

8. Préparations médicamenteuses selon les revendications 1 à 7, caractérisées en ce qu'elles contiennent les composés de formule I en mélange avec des substances auxiliaires et/ou de support usuelles.

9. Utilisation des médicaments selon les revendications 1 à 8 pour la thérapie de néoplasies malignes.

10. Nouveaux composés d'acide oxiranylisocyanurique de formule générale I

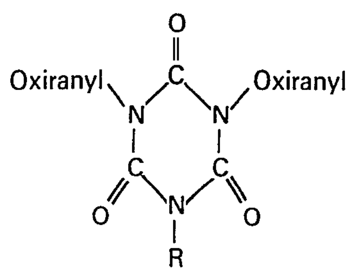

(I)

dans laquelle l'oxiranyle signifie des radicaux identiques ou différents de formule générale II

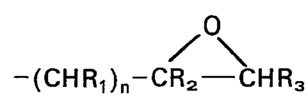

dans laquelle $R_1$, $R_2$ et $R_3$ sont identiques ou différents et signifient de l'hydrogène ou un alcoyle, halogénoalcoyle, hydroxyalcoyle et/ou alcoxyalcoyle contenant chaque fois 1 à 4 atomes de carbone et n est un nombre de 1 à 8, avec comme restriction qu'au cas où, dans tous les radicaux oxiranyle, n soit égal à 1, au moins un des radicaux $R_1$ ou $R_3$ n'est pas de l'hydrogène et qu'en outre R, dans la formule générale I, signifie un radical oxiranyle de formule générale II, de l'hydrogène ou un des radicaux suivants: alcoyle, alcényle, aryle, aralcoyle, alcaryle ou cycloalcoyle ayant un maximum de 12 atomes de carbone.